(19) Europäisches Patentamt
European Patent Office
Office européen des brevets

(11) **EP 4 234 705 A2**

(12) **EUROPÄISCHE PATENTANMELDUNG**

(43) Veröffentlichungstag:
**30.08.2023   Patentblatt 2023/35**

(21) Anmeldenummer: 23168937.3

(22) Anmeldetag: **23.06.2021**

(51) Internationale Patentklassifikation (IPC):
**C12P 5/02** *(2006.01)*

(52) Gemeinsame Patentklassifikation (CPC):
**C12P 5/023;** Y02E 50/30

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(62) Dokumentnummer(n) der früheren Anmeldung(en)
nach Art. 76 EPÜ:
**21181020.5 / 4 108 775**

(71) Anmelder: **Verbio Vereinigte BioEnergie AG
04109 Leipzig (DE)**

(72) Erfinder:
• **BONK,  Fabian
04315 Leipzig (DE)**

• **KÖCKERITZ, Jan
06110 Halle (DE)**
• **SCHLIMBACH, Michael
06120 Halle (Saale) (DE)**
• **LÜDTKE, Oliver
04416 Markkleeberg (DE)**

(74) Vertreter: **Gulde & Partner
Patent- und Rechtsanwaltskanzlei mbB
Wallstraße 58/59
10179 Berlin (DE)**

Bemerkungen:
Diese Anmeldung ist am 20-04-2023 als
Teilanmeldung zu der unter INID-Code 62 erwähnten
Anmeldung eingereicht worden.

(54) **VERFAHREN ZUR BIOLOGISCHEN IN-SITU METHANISIERUNG VON CO2 UND H2 IN EINEM
BIOREAKTOR**

(57)      Die Erfindung betrifft ein Verfahren zur biologischen in-situ Methanisierung von $CO_2$ und $H_2$ in einem Bioreaktor, das dadurch gekennzeichnet ist, dass
a) dem Bioreaktor organisches Substrat zugeführt wird und mindestens ein Teil des organischen Substrates von Mikroorganismen zu Biogas umgewandelt wird,
b) dem Bioreaktor mindestens 0,15 kg Rohfaser pro $m^3$ Bioreaktorvolumen pro Tag zugeführt wird,
c) der Bioreaktor bei 20 - 45 °C betrieben wird,
d) dem Bioreaktor $H_2$ zugeführt wird und mindestens ein Teil des $H_2$ zusammen mit $CO_2$ von Mikroorganismen zu Methan umgewandelt wird.

Fig. 1

EP 4 234 705 A2

**Beschreibung**

Die Erfindung betrifft ein Verfahren zur biologischen in-situ Methanisierung von $CO_2$ und $H_2$ in einem Bioreaktor.

**Technologischer Hintergrund**

Die Umwandlung (Methanisierung) von Kohlenstoffdioxid ($CO_2$) und Wasserstoff ($H_2$) zu Methan ($CH_4$) kann katalytisch, meist nickelbasiert, oder biologisch, d.h. mit Hilfe von Mikroorganismen, erfolgen. Die Methanisierung erfolgt in beiden Fällen nach folgender Gesamtreaktion:

$$CO_2 + 4\,H_2 \rightarrow CH_4 + 2\,H_2O \qquad (1)$$

Im Falle der biologischen Umsetzung wird zudem $CO_2$ und $H_2$ von den beteiligten Mikroorganismen für deren Anabolismus genutzt, z.B. zur Synthese von Enzymen. Die Umsetzung von $CO_2$ und $H_2$ im Anabolismus ist im Vergleich zur Umsetzung zu Methan jedoch gering. Die biologische Methanisierung hat den Vorteil, dass sie weniger anfällig gegen Katalysatorgifte wie Schwefelwasserstoff ($H_2S$) ist, die z.B. in $CO_2$ Strömen aus Biogasanlagen vorkommen können.

Die biologische Methanisierung von $CO_2$ und $H_2$ gliedert sich in die zwei Konzepte ex-situ und in-situ. Beim in-situ Konzept wird einem Bioreaktor $H_2$ zusätzlich zu organischem Substrat zugegeben. Das organische Substrat wird unter anderem zu $CH_4$ und $CO_2$ vergoren. Dieses $CO_2$ wird wiederum mit $H_2$ zu $CH_4$ umgewandelt. Beim ex-situ Konzept wird im Bioreaktor lediglich $CO_2$ und $H_2$, aber kein organisches Substrat umgewandelt. Der Hauptvorteil des in-situ Konzepts ist, dass vorhandene und bisher ausschließlich mit organischem Substrat beschickte Bioreaktoren verwendet werden können, wogegen für ein ex-situ Konzept der kostenintensive Neubau von Bioreaktoren notwendig ist.

Mit Hilfe des in-situ Konzeptes konnten bei thermophiler Temperaturführung (ca. 55°C) $CH_4$-Produktionsraten aus zugeführtem $H_2$ von 0,016 Kubikmeter $CH_4$ pro Kubikmeter Bioreaktorvolumen pro Stunde ($m^3/m^3/h$) erreicht werden (EP2771472B1, Beispiel 1, Tabelle 3). Beim im Patent EP2771472B1 gelehrten in-situ Verfahren mit thermophiler Temperaturführung ist allerdings die Zugabe von saurem Substrat notwendig. Dies ist nachteilig, da saure Substrate nicht überall verfügbar sind und so kostenintensiv über lange Strecken zur Anlage transportiert werden müssen. Auch suggerieren die Ausführungsbeispiele, dass das Verfahren nur für eine geringe Raumbelastung von unter 1,7 kg organischer Trockensubstanz (oTS) in Form von organischem Substrat pro $m^3$ Bioreaktorvolumen pro Tag angewendet werden können. Solche geringen Raumbelastungen anzuwenden ist auch naheliegend, da bekannt ist, dass Prozesse bei thermophiler Temperaturführung eine geringe Prozessstabilität aufweisen.

Dem Problem der geringen Prozessstabilität bei thermophiler Temperaturführung wird im Patent EP2586868B1 dadurch begegnet, dass $H_2$ nur bei einer vorhanden Bioreaktor-Kaskade eingesetzt wird. Bei dieser Kaskade wird der erste Bioreaktor mit organischem Substrat, aber nicht mit $H_2$ beschickt. Der Ablauf des ersten Bioreaktors wird in einen zweiten Bioreaktor gegeben und dieser mit $H_2$ beschickt. Der zweite Bioreaktor wird nur einer geringen Menge an organischem Substrat und damit einer niedrigen Raumbelastung ausgesetzt, da ein Großteil des organischen Substrats bereits im ersten Bioreaktor vergärt wurde. Als Nachteile ergibt sich dadurch allerdings, dass eine Bioreaktor-Kaskade überhaupt vorhanden sein muss, also dieses Verfahren nicht auf einstufige Anlagen angewendet werden kann, und der erste Bioreaktor einer solchen Kaskade für eine $H_2$ Umsetzung nicht zur Verfügung steht.

Darüber hinaus ist ein grundsätzliches Problem der thermophilen Temperaturführung der hohe Energieeinsatz, um die erhöhte Temperatur zu halten. Ein in-situ Konzept bei mesophiler Temperaturführung (ca. 37°C) ist daher zu bevorzugen.

Allerdings wurden mit dem in-situ Konzept unter mesophiler Temperaturführung bisher nur $CH_4$-Produktionsraten aus $CO_2$ und $H_2$ von ca. 0,0017 Kubikmeter an $CH_4$ unter Normbedingungen (273,15 K und 1,01325 bar) pro $m^3$ Bioreaktorvolumen pro Stunde ($Nm^3/m^3/h$) erzielt (DE102012112889A1, [0075]). Als organisches Substrat wurde Überschussschlamm einer Kläranlage mit einem oTS-Gehalt zwischen 2,2 bis 2,8 % zugeführt. Trotz mesophiler Temperaturführung wurde hier nur eine Raumbelastung von 1,25 kg $oTS/m^3/d$ erreicht.

Der im Patentanspruch 1 angegebenen Erfindung liegt das Problem zugrunde, dass beim gegenwärtigen Stand der Technik in einem Bioreaktor mit dem in-situ Konzept unter mesophiler Temperaturführung und bei hoher Raumbelastung von organischem Substrat, die $CH_4$-Produktionsrate durch die Zuführung von $H_2$ nur unzureichend gesteigert werden konnte.

**Zusammenfassung der Erfindung**

1. Bei dieser Erfindung handelt es sich um ein Verfahren zur biologischen in-situ Methanisierung von $CO_2$ und $H_2$

in einem Bioreaktor, dadurch gekennzeichnet, dass

a) dem Bioreaktor organisches Substrat zugeführt wird und mindestens ein Teil des organischen Substrates von Mikroorganismen zu Biogas umgewandelt wird,

b) dem Bioreaktor mindestens 0,15 kg Rohfaser pro $m^3$ Bioreaktorvolumen pro Tag zugeführt wird,

c) der Bioreaktor bei 20 - 45 °C betrieben wird,

d) dem Bioreaktor $H_2$ zugeführt wird und mindestens ein Teil des $H_2$ zusammen mit $CO_2$ von Mikroorganismen zu Methan umgewandelt wird.

Überraschend wurde festgestellt, dass selbst Bioreaktoren mit hoher Raumbelastung ein hohes Potenzial besitzen, $H_2$ umzusetzen. Allerdings sind hohe $CH_4$-Produktionsraten aus zugeführtem $H_2$ nur bei hoher Zufuhr an Rohfasern erreichbar. Dies steht im Gegensatz zur typischen Betriebsweise von Biogasreaktoren, da normalerweise Rohfasern, die v.a. aus schwer abbaubarer Biomasse bestehen, vermieden werden sollen, um zu verhindern, dass sich eine hohe und schwer händelbare Viskosität einstellt, sowie das vorhandene Bioreaktorvolumen nur unzureichend ausgenutzt wird. Allerdings hat es sich bezüglich einer gesteigerten $CH_4$-Produktionsrate aus zugeführtem $H_2$ überraschend als hilfreich erwiesen, eine hohe Menge Rohfasern hinzuzufügen. Dies beruht, ohne Anspruch auf Vollständigkeit und mit der Beschränkung auf eine rein theoretische Betrachtungsweise, auf der Erhöhung der Verweilzeit von Wasserstoffblasen, sowie auf der Vergrößerung der Aufwuchsfläche von Mikroorganismen.

## Detaillierte Beschreibung der Erfindung

Definitionen

Im Zusammenhang der vorliegenden Erfindung wird unter dem Begriff "in-situ" die biologische Methanisierung von zugeführtem $CO_2$ und $H_2$ in einem Bioreaktor verstanden, in dem auch organisches Substrat zu Biogas umgewandelt wird.

Im Zusammenhang der vorliegenden Erfindung wird unter der Trockensubstanz (TS) der Feststoffrückstand verstanden, welcher nach Entfernen des Lösungsmittels (z.B. Wasser) aus einer Suspension (z.B. aus einer Schlempe) oder aus einer Lösung erhalten wird. Das heißt, der Feststoffrückstand ist als Gesamtheit aller zuvor gelösten oder suspendierten Feststoffe (z.B. Rohfasern und Salze) zu verstehen.

Im Zusammenhang der vorliegenden Erfindung wird unter organischer Trockensubstanz (oTS) der Teil des TS verstanden, der bei einer Veraschung des TS nicht als Asche zurückbleibt.

Im Zusammenhang der vorliegenden Erfindung wird unter der Raumbelastung eines Bioreaktors der Massenstrom an oTS bezeichnet, der diesem bezogen auf das Bioreaktorvolumen innerhalb eines Tages durchschnittlich zugeführt wird. Daraus ergibt sich die Einheit kg oTS/$m^3$/d.

Im Zusammenhang der vorliegenden Erfindung werden unter dem Begriff "organisches Substrat" oTS-haltige flüssige oder feste organische Stoffströme verstanden, die dem Bioreaktor zugeführt werden. Darunter fallen unter anderem Nebenprodukte der biomasseverarbeitenden Industrien wie z.B. Schlempe aus einer Bioethanolanlage oder Kartoffelpülpe aus der Stärkeherstellung, sowie land- und forstwirtschaftliche Reststoffe wie beispielsweise Stroh, Haferspelzen, Reisschalen oder Sägespäne. Unter den Begriff "organisches Substrat" fallen nicht die Gase $CO_2$ und $H_2$.

Im Zusammenhang der vorliegenden Erfindung wird unter dem Begriff "Mikroorganismen" eine nicht näher definierte Mischkultur aus Bakterien und/oder Archaeen verstanden, die in typischen Biogasanlagen vorkommen können. Mit "Mikroorganismen" ist nicht eine Reinkultur einer bestimmten Spezies gemeint.

Im Zusammenhang der vorliegenden Erfindung wird zur Beurteilung des Ammoniumgehaltes die Messgröße des wasserdampfflüchtigen Ammoniumstickstoffs ($NH_4$-N) herangezogen. Zu dessen Bestimmung wird Ammoniak aus der Probe mit Hilfe von Wasserdampf ausgetrieben und in einer Borsäurelösung aufgefangen. Anschließend erfolgt eine Titration mit Salzsäure. Über den Verbrauch an Salzsäure kann auf die Konzentration von Ammonium in der Probe geschlossen werden.

Im Zusammenhang der vorliegenden Erfindung wird unter dem Begriff Bioreaktorvolumen das sich im Bioreaktor befindliche Flüssigkeitsvolumen verstanden. Dies bezieht sich ebenfalls auf alle auf das Bioreaktorvolumen normierten Größen wie beispielsweise $CH_4$-Produktionsrate, $H_2$- und $CO_2$-Zufuhrrate. Dieses Bioreaktorvolumen stellt den Ort biochemischer Reaktionen dar.

Im Zusammenhang der vorliegenden Erfindung wird Wasserstoff ($H_2$) in seiner molekularen Form verstanden.

Im Zusammenhang der vorliegenden Erfindung wird unter der $H_2$-Zufuhrrate und der $CO_2$-Zufuhrrate das Volumen an $H_2$ bzw. $CO_2$ unter Normbedingungen (kurz N, 273,15 K und 1,01325 bar) in $m^3$ je $m^3$ Bioreaktorvolumen verstanden, das dem Bioreaktor stündlich durchschnittlich zugeführt wird. Daraus ergibt sich die Einheit N$m^3$/$m^3$/h.

Im Zusammenhang der vorliegenden Erfindung wird unter der $CH_4$-Produktionsrate das Volumen an $CH_4$ unter Normbedingungen (kurz N, 273,15 K und 1,01325 bar) in $m^3$ je $m^3$ Bioreaktorvolumen verstanden, das im Bioreaktor stündlich durchschnittlich gebildet wird. Daraus ergibt sich die Einheit $Nm^3/m^3/h$. In dieser Erfindung wird zwischen $CH_4$-Produktionsrate aus oTS und $CH_4$-Produktionsrate aus $H_2$, der dem Bioreaktor zugeführt wird, unterschieden. Die $CH_4$-Produktionsrate aus oTS entspricht der $CH_4$-Produktionsrate, die im Bioreaktor ohne $H_2$ Zufuhr entsteht. Die $CH_4$-Produktionsrate aus zugeführtem $H_2$ ist in Gleichung 2 definiert

$$CH4 - Produktionsrate\ aus\ zugef\ddot{u}hrtem\ H2 = \frac{H2\ Zufuhrrate - H2\ Ausgangsrate}{4} \qquad (2)$$

Dem Fachmann ist bekannt, dass in der Vergärung von oTS in den Zwischenschritten Acidogenese und Acetogenese $H_2$ gebildet werden kann, der zusammen mit $CO_2$ zu $CH_4$ umgewandelt werden kann. Dieser $H_2$ ist nicht Teil des dem Bioreaktor zugeführtem $H_2$ ($H_2$ Zufuhrrate). Gleichung (2) vernachlässigt den Teil des Wasserstoffs, der von den an der $H_2$-Umwandlung beteiligten Mikroorganismen nicht im Katabolismus zu $CH_4$ umgesetzt wird, sondern im Anabolismus zur Synthese von Molekülen wie z.B. Enzymen verwendet wird.

Im Zusammenhang der vorliegenden Erfindung wird unter dem Begriff "Rohfasergehalt" die in der Futtermittelanalytik nach Weender als "Rohfaser" bezeichneten Fraktion eines organischen Substrates verstanden. Die Messmethode zur Bestimmung des Rohfasergehaltes ist in folgender Quelle beschrieben:

Verband Deutscher Landwirtschaftlicher Untersuchungs- und Forschungsanstalten (VDLUFA) (publisher), 1993: Methode 6.1.1. Bestimmung der Rohfaser (WEENDER-Verfahren). Handbuch der Landwirtschaftlichen Versuchs- und Untersuchungsmethodik (VDLUFA-Methodenbuch), Volume III, Die Untersuchung von Futtermitteln, 3rd Edition, VDLUFA Verlag, Darmstadt.

Die Zufuhr von Rohfasern in den Bioreaktor wird als kg Rohfaser/$m^3$/d angegeben und steht für die Masse an Rohfasern in kg je $m^3$ Bioreaktorvolumen, die dem Bioreaktor an einem Tag durchschnittlich zugeführt werden.

Im Zusammenhang der vorliegenden Erfindung wird unter dem Begriff "Produktgas" das Gas verstanden, das den Bioreaktor in gasförmiger Form verlässt. Produktgas kann aus einer Mischung von gasförmigen Stoffen bestehen, insbesondere $CH_4$, $CO_2$, $H_2$, $H_2S$ und $H_2O$-Dampf. Im Zusammenhang der vorliegenden Erfindung wir unter Biogas ein Gasgemisch verstanden, das beim anaeroben Abbau von organischem Substrat entsteht und vor allem aus $CH_4$ und $CO_2$ besteht.

Im Zusammenhang der vorliegenden Erfindung handelt es sich bei dem Begriff "Produktgas-Aufbereitung" um die teilweise Abtrennung von Gasbestandteilen aus dem Produktgas.

Beschreibung der Erfindung

Bei der vorliegenden Erfindung wird organisches Substrat in einem Bioreaktor bei Temperaturen zwischen 20 - 45°C hauptsächlich zu $CH_4$ und $CO_2$ umgesetzt. Gleichzeitig erfolgt die Zufuhr von $H_2$ in den Bioreaktor, sodass es zur biologischen Umwandlung von $CO_2$ und $H_2$ zu $CH_4$ kommt.

Überraschenderweise wurde festgestellt, dass bei Anwendung des mesophilen Temperaturbereichs (20-45°C) statt des thermophilen Temperaturbereichs wie im Stand der Technik (EP2771472B1, EP2586868B1) die Vorteile überwiegen und sogar höhere $CH_4$-Produktionsraten aus zugeführtem $H_2$ erreicht werden können. Bisher ist man im Stand der Technik davon ausgegangen, dass thermophile Temperaturen zu bevorzugen sind, da bekannt ist, dass die mikrobielle Aktivität bei thermophilen Temperaturen höher als bei mesophilen Temperaturen ist. Mesophile Temperaturen haben den Vorteil höherer Prozessstabilität und einer höheren Energieeffizienz, da weniger Wärmeenergie bereitgestellt werden muss.

Zudem wird in der vorliegenden Erfindung dem Bioreaktor eine hohe Menge an Rohfasern zugeführt. Im Stand der Technik wurden bisher bevorzugt rohfaserarme Substrate wie Molke (EP2771472B1) oder Klärschlamm (EP2586868B1) eingesetzt. Laut Futtermittelanalytik nach Weender versteht man unter dem Rohfasergehalt den Teil eines organischen Substrates, der nach der Behandlung mit verdünnten Säuren und Laugen zurückbleibt, der also nicht leicht "verdaulich" ist. Die Zuführung von Substrat mit hohem Rohfasergehalt in einen Bioreaktor wird daher im Allgemeinen als Nachteil angesehen, da sich die enthaltenen schwer verdaulichen Bestandteile im Bioreaktor akkumulieren können, wodurch sich eine hohe und schwer händelbare Viskosität einstellt und das vorhandene Bioreaktorvolumen nur unzureichend ausgenutzt wird. Probleme mit Schwimmschichten, Rühr- und Pumpprobleme können die Folge sein.

Überraschenderweise wurde festgestellt, dass hinsichtlich einer $H_2$-Zufuhr in einen Bioreaktor die Vorteile einer Zufuhr von einem organischen Substrat oder mehreren organischen Substraten mit hohem Rohfasergehalt die oben beschriebenen Nachteile überwiegen. Dies beruht, ohne Anspruch auf Vollständigkeit und mit der Beschränkung auf eine rein theoretische Betrachtungsweise, auf der Erhöhung der Verweilzeit von Wasserstoffblasen, was deren Transfer in die Reaktorflüssigkeit und deren mikrobielle Umsetzung verbessert, sowie auf der Vergrößerung der Aufwuchsflächen für Mikroorganismen. Auf Grund experimenteller Ergebnisse wurde festgestellt, dass zur Erzielung hoher $CH_4$-Produktionsraten aus zugeführtem $H_2$ mindestens 0,15 kg Rohfaser/$m^3$/d zugeführt werden sollten. Zum Vergleich wird im Stand der Technik bei EP2771472B1 die Zugabe von Molke empfohlen, die praktisch keine Rohfaser enthält (Quelle: https://www.ingredients101.com/whey.htm, abgerufen am 04.05.2021). In einer bevorzugten Ausführungsform das Verfahren so ausgestaltet, dass dem Bioreaktor 0,15 kg Rohfaser/m$^3$/d, bevorzugt 0,2 kg Rohfaser/m$^3$/d, besonders bevorzugt 1,0 kg Rohfaser/m$^3$/d zugeführt werden.

Das Verfahren ist bevorzugt so ausgestaltet, dass ein Großteil der Partikel des organischen Substrats eine Größe von maximal 10 mm aufweist.

Mesophile Mikroorganismen wachsen in einem Temperaturbereich zwischen 20-45°C (Schiraldi und De Rosa, 2014). Allerdings wurde festgestellt, dass die optimale Prozessführung in einem engeren Temperaturbereich liegt. In einer bevorzugten Ausführungsform ist das Verfahren so ausgestaltet, dass der Bioreaktor bei einer Temperatur von 35 - 45°C, bevorzugt bei 36 - 41°C, besonders bevorzugt bei 37 - 39°C betrieben wird.

Bei den sich im Bioreaktor befindlichen Mikroorganismen handelt es sich um eine Mischkultur aus Bakterien und Archaeen. Solche Mischkulturen sind dem Fachmann im Kontext der Biogasproduktion bekannt. Bakterien wandeln in den Prozessschritten Hydrolyse und Acidogenese organisches Substrat unter anderem zu Essig-, Propion- und Buttersäure, $H_2$ und $CO_2$ um. In der Acetogenese wandeln Bakterien Propion- und Buttersäure zu Essigsäure, $H_2$ und $CO_2$ um. In der Methanogenese wandeln Archaeen $H_2$ und $CO_2$, Essigsäure und weitere Methylkomponenten zu Methan um. Abhängig vom Substrat wird in der Methanogenese auch $CO_2$ gebildet. Im Vergleich zu Reinkulturen, wie sie vor allem in ex-situ Konzepten eingesetzt werden und bei denen z.B. nur eine bestimmte methanogene Spezies in einem Bioreaktor kultiviert wird, haben Mischkulturen den Vorteil, dass der Bioreaktor nicht steril betrieben werden muss, um Kontamination mit anderen Mikroorganismen zu vermeiden.

Das Anfahren eines Biogasprozesses ist dem Fachmann bekannt. Zum Beispiel wird zur Inokulation Ablauf aus einer bestehender Biogasanlage oder Tierexkremente verwendet, die bereits eine Mischkultur aus Bakterien und Archaeen enthalten.

In einer bevorzugten Ausführungsform ist das Verfahren so ausgestaltet, dass die hydraulische Verweilzeit (HRT) mindestens 18 Tage, bevorzugt mindestens 25 Tage, besonders bevorzugt mindestens 35 Tage aufweist.

In einer bevorzugten Ausführungsform ist das Verfahren so ausgestaltet, dass die $H_2$-Zufuhr mindestens 0,017 Nm$^3$/m$^3$/h, bevorzugt 0,125 Nm$^3$/m$^3$/h, besonders bevorzugt 0,167 Nm$^3$/m$^3$/h beträgt. In alternativen Verfahren im Stand der Technik wurden bisher unter mesophiler Temperaturführung nur 0,015 Nm$^3$/m$^3$/h (DE102012112889, [0075]) und unter thermophiler Temperaturführung 0,118 Nm$^3$/m$^3$/h erreicht (EP2771472B1, [0102], berechnet aus 1,7 Nl/d und 0,6 L Bioreaktorvolumen). Somit besteht die Möglichkeit mit Hilfe der beschriebenen Erfindung die $H_2$-Zufuhr Vergleich zum gegenwärtigen Stand der Technik zu steigern, somit die $CH_4$-Produktionsraten zu erhöhen und dadurch die Produktivität von Bioreaktoren zu verbessern.

In einer bevorzugten Ausführungsform ist das Verfahren so ausgestaltet, dass die Rohfasern in Form von organischem Substrat, besonders bevorzugt in Form von organischem Substrat pflanzlichen Ursprungs zugeführt werden. Somit wird der Einsatz kostengünstiger organischer Substrate, z.B. Reststoffe wie Stroh, ermöglicht.

In einer bevorzugten Ausführungsform ist das Verfahren so ausgestaltet, dass dem Bioreaktor, trotz $H_2$-Zufuhr, das organische Substrat mit einer Raumbelastung von mehr als 2,5 kg oTS/m$^3$/d, besonders bevorzugt mehr als 3,0 kg oTS/m$^3$/d, zugeführt wird. Im gegenwärtigen Stand der Technik wurde die Zuführung von $H_2$ in einen Bioreaktor nur bei einer geringen Raumbelastung angewendet, z.B. 2,1 kg oTS/m$^3$/d im Patent EP2586868A2 oder 1,7 kg oTS/m$^3$/d im Patent EP2771472B1. Für das Patent EP2586868A2 erfolgte die Berechnung auf Grundlage der angegebenen maximalen $CH_4$-Produktionsrate aus oTS (EP2586868A2, [0034]) und einem üblichen Gasertrag für Klärschlamm von 300 Nl/kg oTS. Die Berechnung der Raumbelastung aus Patent EP2771472B1 beruht aus der angegebenen Verweilzeit, der Reaktorgröße und den angegebenen Stoffdaten des organischen Substrates (EP2771472B1, [0101] und [0102]).

In einer bevorzugten Ausführungsform ist das Verfahren so ausgestaltet, dass der dem Bioreaktor zugeführte $H_2$ aus der Elektrolyse von Wasser stammt. In einer besonders bevorzugten Ausführungsform ist das Verfahren so ausgestaltet, dass der Strom für die Elektrolyse aus regenerativen Quellen erzeugt wurde. Dies hat den Vorteil, dass das im Bioreaktor aus dem Elektrolyse-$H_2$ erzeugte $CH_4$ ein nachhaltiger, strombasierter Kraftstoff ist, der unabhängig von Erdöl und Erdgas gewonnen wurde.

Falls der zugeführte $H_2$ im Bioreaktor nicht komplett umgesetzt wird, verlässt er den Bioreaktor im Produktgas. Das Verfahren kann so ausgestaltet werden, dass ein Teil des Produktgases dem Bioreaktor zugeführt wird. In einer bevorzugten Ausführungsform ist das Verfahren so ausgestaltet, dass das aus dem Bioreaktor austretende Gasgemisch, das Produktgas, einer Produktgas-Aufbereitung zugeführt wird, in der mindestens ein Teil des im Produktgas befindlichen $H_2$ abgetrennt und in den Bioreaktor zurückgeführt wird. Dadurch kann die $CH_4$-Produktionsrate gesteigert und der $H_2$-Gehalt im aufbereiteten Produktgas reduziert werden. In einer besonders bevorzugten Ausführungsform ist das Verfahren so ausgestaltet, dass die Abtrennung des $H_2$ in einer Membrananlage erfolgt. In einer besonders bevorzugten Ausführungsform ist das Verfahren so ausgestaltet, das der abgetrennte und zurückgeführte $H_2$-haltige Gasstrom maximal 90 % (v/v) $H_2$ enthält. Den $H_2$-Gehalt zu beschränken ist vorteilhaft, da so einfache Aufbereitungsanlagen, z.B. einstufige Membrananlagen, zum Einsatz kommen können und so im Vergleich zur Abtrennung von fast reinem $H_2$ energiesparend gearbeitet wird.

In einer bevorzugten Ausführungsform ist das Verfahren so ausgestaltet, dass dem Bioreaktor $CO_2$ zugeführt wird. Dadurch wird in einer bevorzugten Ausführungsform der pH-Wert auf unter 8,2 reguliert. Dies trägt maßgeblich zur Verbesserung der Prozessstabilität bei. In einer bevorzugten Ausführungsform ist das Verfahren so ausgestaltet, dass das zugeführte $CO_2$ aus einer Bioethanolanlage stammt. Das ist vorteilhaft, da sonst die $CH_4$-Produktionsrate aus zugeführtem $H_2$ über die Menge an $CO_2$ limitiert wäre, die aus dem organischen Substrat im Bioreaktor produziert wird. Die Nutzung von $CO_2$ aus einer Bioethanolanlage ist besonders vorteilhaft, da dieses $CO_2$ ursprünglich aus der Atmosphäre beim Pflanzenwachstum mit Hilfe von Sonnenlicht als erneuerbare Energie gebunden wurde und während der Fermentation in konzentrierter Form als Gas mit über 90 % (v/v) $CO_2$-Gehalt anfällt. Das $CO_2$ mit technischen Mitteln stattdessen aus der Atmosphäre oder Verbrennungsgasen zu gewinnen, würde hohe Energie- und Betriebskosten verursachen, da dort das $CO_2$ in viel geringerer Konzentration anfällt, z.B. 0,04 % in der Atmosphäre. In einer bevorzugten Ausführungsform ist das Verfahren so ausgestaltet, dass das Produktgas einer Produktgas-Aufbereitung zugeführt wird, in der mindestens ein Teil des im Produktgas befindlichen $CO_2$ abgetrennt und in den Bioreaktor zurückgeführt wird.

In einer bevorzugten Ausführungsform ist das Verfahren so ausgestaltet, dass die Konzentration von $CO_2$ im Produktgas einen Wert von 5 % (v/v), bevorzugt 10 % (v/v), besonders bevorzugt 40 % (v/v), bezogen auf das Produktgas ohne den Wasseranteil, nicht unterschreitet. Im Stand der Technik wird man von solch hohen $CO_2$-Gehalten weggeleitet, da es normalerweise das Ziel ist, den $CO_2$-Gehalt im Produktgas so weit wie möglich zu senken, am besten auf kleiner 5 % (v/v), um das Produktgas direkt in bestehende Erdgasnetze einspeisen zu können (z.B. EP2771472B1 [0067], EP2586868A2 [0013] und DE102012112889A1 [0041]). Überraschend wurde festgestellt, dass durch hohe $CO_2$-Konzentrationen die Gesamteffektivität des Prozesses verbessert werden kann. Zwar wird der Aufwand der Gasreinigung durch die höheren $CO_2$-Konzentrationen im Produktgas erhöht, doch überwiegt der Vorteil einer höheren Produktivität des Bioreaktors. Im Bioreaktor können so eine deutlich höhere Prozessstabilität, insbesondere geringe pH-Schwankungen und niedrige Konzentrationen an organischen Säuren erreicht werden. Dadurch kann eine hohe Raumbelastung des Bioreaktors genutzt werden, welches eine Steigerung der $CH_4$-Produktionsrate und eine optimale Ausnutzung des zur Verfügung stehenden Gärvolumens zur Folge hat. In einer bevorzugten Ausführungsform ist das Verfahren so ausgestaltet, dass die Konzentration von $CO_2$ im Produktgas durch die Menge an dem Bioreaktor zugeführtem $CO_2$ reguliert wird. In einer weiteren Ausführungsform ist das Verfahren so ausgestaltet, dass die Regulierung der Konzentration von $CO_2$ im abgehenden Produktgas durch die Menge an dem Bioreaktor zugeführtem $H_2$ eingestellt wird.

In einer bevorzugten Ausführungsform ist das Verfahren so ausgestaltet, dass dem Bioreaktor pro Mol zugeführtem $H_2$ mindestens 0,3 mol $CO_2$, besonders bevorzugt mindestens 0,5 mol $CO_2$ zugeführt werden. Dies hat den Vorteil, dass der $CO_2$ Gehalt im Produktgas auf eine hohe Konzentration eingestellt wird. Der Stand der Technik lehrt das Gegenteil, nämlich gar kein $CO_2$ oder maximal die stöchiometrische Menge (0,25 mol $CO_2$ pro mol $H_2$ zuzuführen), um ein Produktgas mit möglichst niedrigem $CO_2$-Gehalt zu erreichen. Dies hat allerdings den Nachteil von Prozessinstabilität.

In einer bevorzugten Ausführungsform ist das Verfahren so ausgestaltet, dass das dem Bioreaktor zugeführte $CO_2$ eine Reinheit von mindestens 90 %, besonders bevorzugt 95 % aufweist. Dadurch wird die benötigte zugeführte Gasmenge minimiert, sowie die Regelung des pH-Wertes im Bioreaktor vereinfacht.

In einer bevorzugten Ausführungsform ist das Verfahren so ausgestaltet, dass die dem Bioreaktor zugeführten Gase nahe am Boden des Bioreaktors zugeführt werden. Das hat den Vorteil, dass das Gas eine längere Verweilzeit im Bioreaktorvolumen hat. In einer weiteren Ausführungsform sind die Bioreaktoren mit Rührwerken ausgestattet und die Zuführung von Gasen erfolgt nahe der Rührwerke. Das hat den Vorteil, dass der Kontakt zwischen Gas und Flüssigkeit erhöht wird.

In einer bevorzugten Ausführungsform ist das Verfahren so ausgestaltet, dass der Bioreaktor der erste Bioreaktor in einer Kaskade von Bioreaktoren ist. Diese Ausgestaltung ist besonders vorteilhaft gegenüber dem Stand der Technik EP2586868B1, bei dem der erste Bioreaktor einer Bioreaktor-Kaskade auf Grund einer geringen Prozessstabilität, nicht zur Zufuhr von $H_2$ geeignet ist. Überraschenderweise wurde in der Erfindung festgestellt, dass die hohe Zufuhr von Rohfasern, die Zufuhr von ammoniumarmer Flüssigkeit und die Zufuhr von $CO_2$ diese Probleme der Prozessstabilität beheben können und so das Bioreaktorvolumen des ersten Bioreaktors für die Umsetzung von $H_2$ verfügbar machen.

In einer bevorzugten Ausführungsform ist das Verfahren so ausgestaltet, dass die $NH_4$-N Konzentration im Bioreaktor abgesenkt wird. In einer bevorzugten Ausführungsform ist das Verfahren so ausgestaltet, dass der $NH_4$-N Gehalt im Bioreaktor auf maximal 6000 mg/kg eingestellt wird. Diese Senkung des $NH_4$-N Gehalts ist vorteilhaft, da so auch die Konzentration an für die Prozessstabilität besonders schädlichen Ammoniak ($NH_3$) im Bioreaktor gesenkt wird. $NH_3$ steht im Säure-Base-Gleichgewicht mit Ammonium ($NH_4^+$) und der Anteil an $NH_3$ steigt mit steigendem pH-Wert. Bei unzureichender $CO_2$ Zufuhr in den Bioreaktor kann der pH-Wert im Bioreaktor durch die Umwandlung von $CO_2$ zu $CH_4$ steigen und so das $NH_4^+/NH_3$ Gleichgewicht mit negativen Konsequenzen in Richtung des $NH_3$ verschieben. In einer weiteren Ausführungsform ist das Verfahren so ausgestaltet, dass die Einstellung des $NH_4$-N Gehalts durch die Auswahl des dem Bioreaktor zugeführten organischen Substrats erfolgt. Zum Beispiel kann ein stickstoffarmes Substrat ausgewählt werden, dass dem Bioreaktor zusätzlich zu einem stickstoffreichen organischen Substrat zugeführt wird.

In einer bevorzugten Ausführungsform ist das Verfahren so ausgestaltet, dass die Senkung des $NH_4$-N Gehaltes durch die Zugabe einer ammoniumarmen Flüssigkeit erfolgt. Dies kann z.B. durch die Zugabe von Frischwasser umgesetzt werden. Dabei würde es zu einem kostenintensiven Verbrauch an Frischwasser kommen. Deshalb ist das Verfahren in einer bevorzugten Ausführungsform so ausgestaltet, dass die ammoniumarme Flüssigkeit durch eine Behandlung des Ablaufs des Bioreaktors, insbesondere durch eine Ammoniumstrippung, bereitgestellt wird. Somit werden durch einen vergleichsweise geringen Investitions- und Betriebskostenaufwand für eine Ablaufbehandlung die deutlich höheren mit einer Frischwasserzufuhr verbundenen Kosten vermieden.

In einer bevorzugten Ausführungsform ist das Verfahren so ausgestaltet, dass es sich beim Bioreaktor um einen Rührkesselreaktor handelt. In einer besonders bevorzugten Ausführungsform ist das Verfahren so ausgestaltet, dass der Bioreaktor kontinuierlich oder semi-kontinuierlich betrieben wird. In einer bevorzugten Ausführungsform ist das Verfahren so ausgestaltet, dass der Bioreaktor keine anorganischen Füll- oder Aufwuchskörper beinhaltet.

In einer bevorzugten Ausführungsform befindet sich im Gasraum des Bioreaktors nur ein geringer Überdruck von weniger als 150 mbar. Dadurch wird eine kostensparende Bauform, Wartung und eine hohe Arbeitssicherheit gewährleistet. Gleichzeitig wird der Aufwand zur Druckerzeugung zur Eduktzufuhr in den Bioreaktor sowie zur Entspannung zur Abführung des Produktgases minimiert.

## Kurzbeschreibung der Figuren

Die Erfindung wird nachfolgend anhand von vier Ausführungsbeispielen und dazugehörigen Zeichnungen näher erläutert.

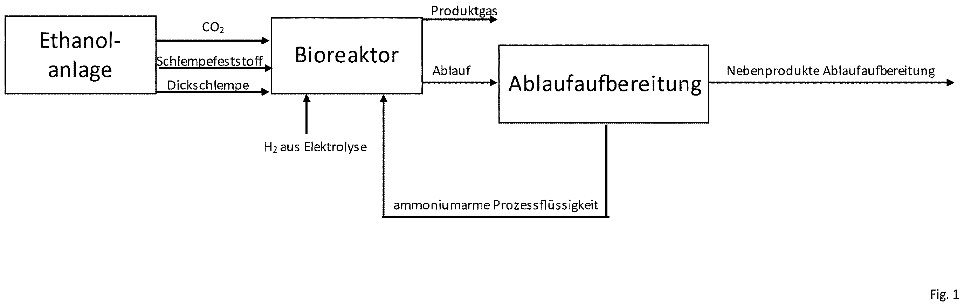

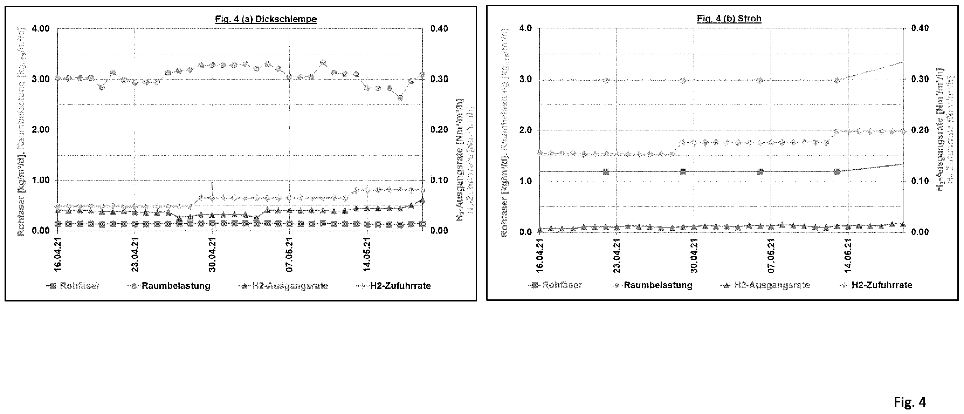

Fig. 1, Fig. 2 und Fig.3 beschreiben den schematischen Prozessablauf von erfindungsgemäßen Ausführungsformen des Verfahrens. Die Fig. 4 (a) und 4 (b) geben Diagramme experimenteller Daten wieder.

## Ausführungsbeispiel 1

Fig. 1 zeigt eine schematische Darstellung des Verfahrens in einer Ausführungsform mit $CO_2$-Zufuhr aus einer Ethanolanlage in den Bioreaktor sowie einer Ablaufaufbereitung mit Ammoniumstrippung und Rückführung der dabei entstehenden ammoniumarmen Prozessflüssigkeit zur Einstellung des $NH_4$-N Gehalts im Bioreaktor. Tabelle 1 zeigt Flüssigkeits- und Feststoffströme, wie z.B. die Massenströme an organischem Substrat in Form der Raumbelastung, Rohfaser und ammoniumarmer Prozessflüssigkeit. Tabelle 2 zeigt die Volumenströme der dem Biorektor zugeführten und vom Bioreaktor abgeführten Gasen.

In diesem und den folgenden Beispielen werden die den Biorektor verlassenden Volumenströme an $H_2$, $CO_2$ und $H_2S$ als $H_2$-, $CO_2$- bzw. $H_2S$-Ausgangsraten bezeichnet und beinhalten das Volumen des jeweiligen Gases unter Normbedingungen (kurz N, 273,15 K und 1,01325 bar) in $m^3$ je $m^3$ Bioreaktorvolumen, das aus dem Bioreaktor stündlich durchschnittlich abgeführt wird. Daraus ergibt sich die Einheit $Nm^3/m^3/h$. Unter dem Begriff "Schlempe" wird der Rückstand aus der Destillation einer ethanolhaltigen Getreidemaische verstanden. Der Begriff "Dickschlempe" wird synonym für Schlempe verwendet. Im Zusammenhang der vorliegenden Erfindung wird unter "Schlempefeststoff" die feste Phase verstanden, die durch eine Fest-Flüssig-Trennung aus der Schlempe abgetrennt wird.

## Schritt 1:

Einem Bioreaktor mit 1000 $m^3$ Bioreaktorvolumen werden 11207 kg Dickschlempe pro Tag aus einer Bioethanolanlage als organisches Substrat zugeführt. Der TS-Gehalt der Dickschlempe beträgt 0,19 kg TS pro kg Dickschlempe. Der oTS-Gehalt der Dickschlempe beträgt 0,91 kg oTS pro kg TS. Damit ergibt sich eine Zufuhr an Dickschlempe von 1937 kg oTS pro Tag bzw. auf das Bioreaktorvolumen des Bioreaktors bezogen eine Raumbelastung von 1,94 kg $oTS/m^3/d$.

Beruhend auf der Futtermittelanalytik nach Weender beträgt der Rohfaser-Gehalt der Dickschlempe 0,039 kg Rohfaser pro kg TS. Berechnet über den TS-Gehalt der Dickschlempe und den Massenstrom an zugeführter Dickschlempe ergibt sich eine sehr geringe Zufuhr an Rohfasern über die Dickschlempe von nur 0,08 kg $Rohfaser/m^3/d$.

Da überraschend festgestellt wurde, dass ein deutlich höherer Rohfasergehalt die $CH_4$-Produktionsrate aus zugeführtem $H_2$ begünstigt, wird zusätzlich zur Dickschlempe dem Bioreaktor 4065 kg/d Schlempefeststoff zugeführt. Schlempefeststoff weist einen hohen TS-Gehalt von ca. 0,33 kg TS pro kg Schlempefeststoff und einen oTS Gehalt von 0,96 kg oTS pro kg TS auf und wird typischerweise als Futtermittel verkauft. Mit einem hohen Rohfaser-Gehalt von 0,14 kg Rohfaser pro kg TS ist Schlempefeststoff kein typisches organisches Substrat für die Biogasproduktion. In diesem Ausführungsbeispiel jedoch erweist er sich als sehr nützlich, um die Rohfaserzufuhr um 0,19 kg $Rohfaser/m^3/d$ deutlich zu erhöhen.

Dem Bioreaktor werden zudem 6000 kg/d gereinigter Ablauf aus Schritt 3, 15,8 $Nm^3$ $CO_2/h$ aus einer Bioethanolanlage und 25 $Nm^3$ $H_2/h$ aus einer Elektrolyse zugeführt.

Tabelle 1 Flüssigkeits- und Feststoffströme Ausführungsbeispiel 1

|  | Zahlenwert | Einheit |
|---|---|---|
| Raumbelastung Dickschlempe | 1,9 | kg oTS/m$^3$/d |
| Raumbelastung Schlempefeststoff | 1,3 | kg oTS/m$^3$/d |
| Rohfaser Dickschlempe | 0,08 | kg Rohfaser/m$^3$/d |
| Rohfaser Schlempefeststoff | 0,19 | kg Rohfaser/m$^3$/d |
| Ammoniumarme Prozessflüssigkeit aus Schritt 3 | 6 | kg/m$^3$/d |

**Schritt 2:**

Organisches Substrat wird im Bioreaktor unter anderem zu den Gasen $CH_4$, $CO_2$ und $H_2S$ umgesetzt. Der aus der Elektrolyse zugeführte $H_2$ wird zu ca. 60 % umgesetzt. Der restliche $H_2$ verlässt den Bioreaktor mit den anderen Gasen über das Produktgas (siehe Tabelle 2).

Die Zufuhr von $CO_2$ aus der Bioethanolanlage führt dazu, dass der $CO_2$ Gehalt im Produktgas ca. 45 % (v/v) bezogen auf Produktgas ohne den Wasseranteil beträgt. Ohne $CO_2$ Zufuhr aus der Bioethanolanlage würde der $CO_2$ Gehalt im Produktgas nur ca. 37 % (v/v) bezogen auf Produktgas ohne den Wasseranteil betragen, was zu einem unerwünschten, höheren pH-Wert führen würde. Durch die Zufuhr von $CO_2$ aus der Bioethanolanlage ergibt sich so eine stabilere Reaktionsführung im Bioreaktor.

Die Temperatur im Bioreaktor wird auf 37 °C geregelt.

Im hier beschriebenen Ausführungsbeispiel würde ohne $H_2$- und $CO_2$-Zuführung eine $CH_4$-Produktionsrate von 0,053 Nm$^3$/m$^3$/h erreicht werden. Mit Hilfe der beschriebenen Erfindung erhöht sich die $CH_4$-Produktionsrate durch die Zuführung und Umwandlung von $H_2$ und $CO_2$ auf 0,056 Nm$^3$/m$^3$/d. Die $CH_4$-Produktionsrate aus zugeführtem $H_2$ beträgt 0,0038 Nm$^3$/m$^3$/h. Somit wird die Ausnutzung des zur Verfügung stehenden Bioreaktorvolumens gesteigert.

Tabelle 2 Gasströme Ausführungsbeispiel 1

|  | Zahlenwert | Einheit |
|---|---|---|
| **Gaszufuhrraten** |  |  |
| $H_2$ aus Elektrolyse | 0,025 | Nm$^3$/m$^3$/h |
| $CO_2$ aus Bioethanolanlage | 0,016 | Nm$^3$/m$^3$/h |
| **Produktgas** |  |  |
| $CH_4$-Produktionsrate | 0,056 | Nm$^3$/m$^3$/h |
| $CO_2$-Ausgangsrate | 0,055 | Nm$^3$/m$^3$/h |
| $H_2$-Ausgangsrate | 0,010 | Nm$^3$/m$^3$/h |
| $H_2S$-Ausgangsrate | 0,001 | Nm$^3$/m$^3$/h |

**Schritt 3:**

Der Ablauf des Bioreaktors wird einer Ablaufaufbereitung mit Ammoniumstrippung zugeführt und Ammonium zum Großteil entfernt. Die dabei entstehende ammoniumarme Prozessflüssigkeit weist einen $NH_4$-N Gehalt von 500 mg/kg auf. Ein Teil dieser ammoniumarmen Prozessflüssigkeit dem Bioreaktor zur Einstellung des $NH_4$-N Gehaltes zugeführt und damit der $NH_4$-N Gehalt auf unter 6000 mg/kg eingestellt.

**Ausführungsbeispiel 2**

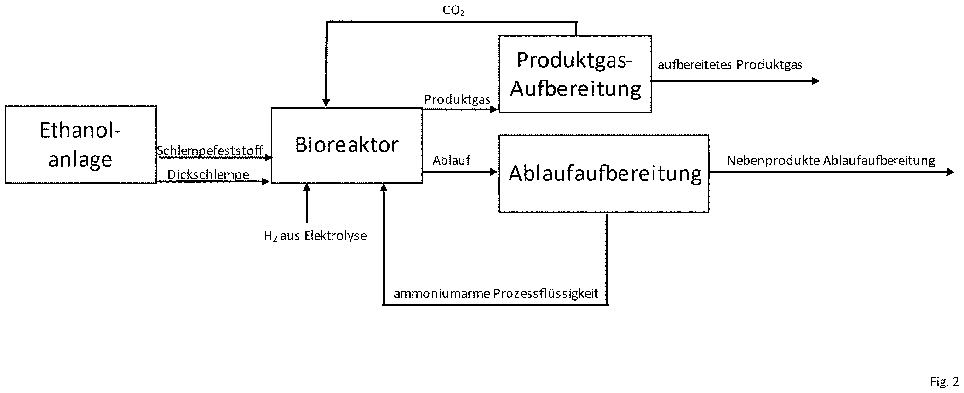

Eine weitere Möglichkeit der Prozessführung besteht darin, statt auf $CO_2$ aus einer Bioethanolanlage auf das $CO_2$ zuzugreifen, das bei der Biogasproduktion aus organischem Substrat entsteht. Dazu wird dem in Ausführungsbeispiel 1 beschriebenen Verfahren eine Produktgas-Aufbereitung hinzugefügt, in der $CO_2$ mit einer Kreislaufrate von 0,016 Nm$^3$ $CO_2$ pro m$^3$ Bioreaktorvolumen pro Stunde aus dem Produktgas abgetrennt und zurück in den Bioreaktor geführt wird. Dieses $CO_2$ ersetzt den $CO_2$ Strom aus der Bioethanolanlage in Ausführungsbeispiel 1. Fig. 2 zeigt eine schematische Darstellung dieses Verfahrens. Tabelle 3 zeigt die Gasströme. Die Flüssigkeitsströme in diesem Beispiel sind gleich zu Ausführungsbeispiel 1 und in Tabelle 1 dargestellt. Die Temperatur im Bioreaktor wird weiterhin auf 37 °C geregelt und die Ablaufbehandlung aus Schritt 3 in Ausführungsbeispiel 1 beibehalten.

Kreislaufrate meint hier und im Folgenden das Volumen des jeweiligen Gases unter Normbedingungen (kurz N, 273,15 Kund 1,01325 bar) in $m^3$ je $m^3$ Bioreaktorvolumen, welches in der Produktgas-Aufbereitung teilweise aus dem Produktgas abgetrennt und pro Stunde durchschnittlich zurück in den Bioreaktor geleitet wird. Daraus ergibt sich die Einheit $Nm^3/m^3/h$.

Wie auch in Ausführungsbeispiel 1 ist der $CO_2$-Gehalt im Produktgas 45 % (v/v) bezogen auf Produktgas ohne den Wasseranteil und damit vorteilhaft höher, als er mit 37 % (v/v) bezogen auf Produktgas ohne den Wasseranteil ohne $CO_2$-Zufuhr wäre.

Da die veränderte $CO_2$-Quelle bei sonst gleichbleibenden Betriebsbedingungen wie in Ausführungsbeispiel 1 keinen Einfluss auf die $CH_4$-Produktionsrate hat, beträgt die $CH_4$-Produktionsrate aus zugeführtem $H_2$ ebenfalls 0,0038 $Nm^3/m^3/h$.

Tabelle 3 Gasströme Ausführungsbeispiel 2

|  | Zahlenwert | Einheit |
|---|---|---|
| **Gaszufuhrraten** |  |  |
| $H_2$ aus Elektrolyse | 0,025 | $Nm^3/m^3/h$ |
| $CO_2$ aus Produktgas-Aufbereitung | 0,016 | $Nm^3/m^3/h$ |
| **Produktgas** |  |  |
| $CH_4$ | 0,056 | $Nm^3/m^3/h$ |
| $CO_2$-Ausgangsrate | 0,055 | $Nm^3/m^3/h$ |
| $H_2$-Ausgangsrate | 0,010 | $Nm^3/m^3/h$ |
| $H_2$S-Ausgangsrate | 0,001 | $Nm^3/m^3/h$ |
| **Produktgas-Aufbereitung** |  |  |
| $CO_2$-Kreislaufrate | 0,016 | $Nm^3/m^3/h$ |
| Aufbereitetes Produktgas |  |  |
| • $CH_4$-Produktionsrate | 0,056 | $Nm^3/m^3/h$ |
| • CO2 | 0,039 | $Nm^3/m^3/h$ |
| • H2 | 0,010 | $Nm^3/m^3/h$ |
| • H2S | 0,001 | $Nm^3/m^3/h$ |

**Ausführungsbeispiel 3**

Wenn eine Einspeisung von Produktgas in das Erdgasnetz geplant ist, müssen bestimmte Regeln des Netzbetreibers hinsichtlich der Zusammensetzung des Gases eingehalten werden. Als Regel kann z.B. gelten, dass der $CH_4$-Gehalt größer 95 % (v/v), der $H_2$-Gehalt kleiner 2 % (v/v) und der $H_2$S-Gehalt ca. 0 % (v/v) bezogen auf Gas ohne den Wasseranteil sein muss.

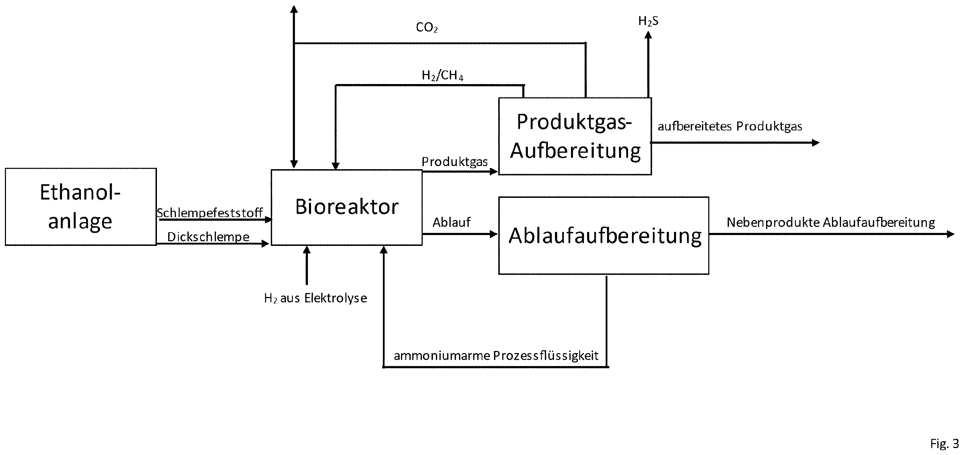

Fig.3 zeigt eine schematische Darstellung eines abgewandelten Verfahrens nach Ausführungsbeispiel 2 mit ausgeprägterer Produktgas-Aufbereitung und Gasrückführungen in den Bioreaktor. $H_2$S wird fast vollständig aus dem Produktgas entfernt. Ein Großteil des $CO_2$ wird aus dem Produktgas entfernt und ein Teil davon in den Bioreaktor zurückgeführt und der Rest einer anderen Verwertung zugeführt. $H_2$ wird über eine Membran abgetrennt. Hinsichtlich Invest- und Betriebskosten wird hier vorteilhaft nur eine Trennstufe eingesetzt. Diese reicht aus, um eine typische $H_2$-Maximumkonzentration von 2 % (v/v) für die Gaseinspeisung in ein Erdgasnetz einzuhalten, führt allerdings dazu, dass mit dem $H_2$ auch ein Teil $CH_4$ wieder in den Bioreaktor zurückgeführt wird.

Tabelle 4 zeigt die Gasströme in diesem Ausführungsbeispiel. Die Flüssigkeitsströme in diesem Beispiel sind gleich zu Ausführungsbeispiel 1 und in Tabelle 1 dargestellt. Die Temperatur im Bioreaktor wird weiterhin auf 37 °C geregelt und die Ablaufbehandlung aus Schritt 3 in Ausführungsbeispiel 1 beibehalten.

Durch die Rückführung von $H_2$ wird die Verweilzeit des $H_2$ im Bioreaktor erhöht. Dadurch kann der Umsatz des aus dem Elektrolyseur stammenden $H_2$ von 60 % auf 95 % (v/v) erhöht werden. Die $CH_4$-Produktionsrate aus

zugeführtem $H_2$ beträgt damit 0,00594 $Nm^3/m^3$/h. Durch die erhöhten Mengen an rückgeführtem Gas ist auch die Erhöhung der Rückführung an $CO_2$ nötig, um den $CO_2$-Gehalt im Produktgas weiterhin hochzuhalten. Dadurch können die in Ausführungsbeispiel 1 beschriebenen Vorteile einer stabilen $CO_2$-Konzentration im Produktgas beibehalten werden.

Tabelle 4 Gasströme Ausführungsbeispiel 3

| | Zahlenwert | Einheit |
|---|---|---|
| **Gaszufuhrraten** | | |
| $H_2$-Zufuhrrate (aus Elektrolyse) | 0,025 | $Nm^3/m^3$/h |
| $CO_2$ aus Produktgas-Aufbereitung | 0,028 | $Nm^3/m^3$/h |
| $H_2$-Zufuhrrate (aus Produktgas-Aufbereitung) | 0,015 | $Nm^3/m^3$/h |
| $CH_4$ aus Produktgas-Aufbereitung | 0,004 | $Nm^3/m^3$/h |
| **Produktgas** | | |
| $CH_4$ | 0,063 | $Nm^3/m^3$/h |
| $CO_2$-Ausgangsrate | 0,065 | $Nm^3/m^3$/h |
| $H_2$-Ausgangsrate | 0,016 | $Nm^3/m^3$/h |
| $H_2S$-Ausgangsrate | 0,001 | $Nm^3/m^3$/h |
| **Produktgas-Aufbereitung** | | |
| $CO_2$-Kreislaufrate | 0,028 | $Nm^3/m^3$/h |
| $CO_2$ zu anderer Verwertung | 0,034 | $Nm^3/m^3$/h |
| $H_2$-Kreislaufrate | 0,015 | $Nm^3/m^3$/h |
| $CH_4$-Kreislaufrate | 0,004 | $Nm^3/m^3$/h |
| $H_2S$ | 0,001 | $Nm^3/m^3$/h |
| Aufbereitetes Produktgas | | |
| • $CH_4$-Produktionsrate | 0,058 | $Nm^3/m^3$/h |
| • $CO_2$ | 0,002 | $Nm^3/m^3$/h |
| • $H_2$ | 0,001 | $Nm^3/m^3$/h |
| • $H_2S$ | 0,000 | $Nm^3/m^3$/h |

**Ausführungsbeispiel 4**

Ausführungsbeispiel 4 beinhaltet Experimente im Technikumsmaßstab.

*Methode*

Zwei Bioreaktoren "A" und "B" des Typs Rührkesselreaktor wurden mit verschiedenen organischen Substraten sowie $H_2$ und $CO_2$ beschickt.

Bioreaktor A wurde mit Dickschlempe und ammoniumarmer Prozessflüssigkeit aus einer Bioethanolanlage gefüttert. Die Fütterung erfolgte semi-kontinuierlich in 5 gleichgroßen Intervallen pro Tag verteilt.

Bioreaktor B wurde mit Getreidestroh und ammoniumarmer Prozessflüssigkeit gefüttert. Die Fütterung erfolgte einmal täglich.

Das Bioreaktorvolumen betrug 75 l (Bioreaktor A) und 55 l (Bioreaktor B). Die Durchmischung erfolgte mit zentralen Rührwerken mit einer Umdrehungsgeschwindigkeit von 370 rpm bei beiden Bioreaktoren. Die Regulierung der Temperatur des Bioreaktorvolumens wurde mit Hilfe von Wasser als Heizmedium mit ca. 39 °C realisiert, das die Bioreaktoren über einen Doppelmantel umströmte. Die zugeführten Mengen an $H_2$ und $CO_2$ wurden für jedes Gas separat über thermische Massendurchflussregler (EL-Flow Select, Bronkhorst Deutschland Nord GmbH) geregelt. Produktgasvolumenströme wurden in beiden Bioreaktoren mit Trommel-Gaszählern (TG 0.5, Dr.-Ing. RITTER Apparatebau GmbH & Co. KG) ermittelt. Die Bestimmung der Gaszusammensetzung des Produktgases erfolgte einmal wöchentlich mit Hilfe eines Gas-GC (MobilGC, ECH Elektrochemie Halle GmbH; Konfiguration: Hayesep QS Säule 45 °C, Molsiebsäule 55 °C, Wärmeleitfähigkeitsdetektor, Trägergas Argon). Der TS-Gehalt der Substrate wurde gravimetrisch nach Trocknung bei 105°C bis zur Massekonstanz bestimmt. Der Aschegehalt wurde gravimetrisch nach Ausglühen bei 650 °C über mindestens 2 h bestimmt. Der oTS-Gehalt wurde als Differenz zwischen TS-Gehalt und Aschegehalt berechnet. Der Rohfasergehalt wurde nach VDLUFA-Methodenbuch bestimmt (siehe Abschnitt Definitionen). Für die Bestimmung der Raumbelastung und der zugeführten Rohfaser wurde die ammoniumarme Prozessflüssigkeit vernachlässigt.

*Ergebnisse und Diskussion*

Figure 4 zeigt Daten der beiden Bioreaktoren von 16.04.2021 bis 19.05.2021. Davor wurden beide Reaktoren bereits über ein Jahr betrieben, so dass transiente Effekte der Start-Up Phase ausgeschlossen und bei der Interpretation der Daten vernachlässigt werden können.

In beiden Bioreaktoren wird organisches Substrat von Mikroorganismen zu Biogas umgewandelt, was an einer niedrigeren Menge an oTS im Ablauf im Vergleich zum Zulauf der Reaktoren, sowie an den gemessenen $CH_4$-Produk-tionsraten zu erkennen ist, die höher als die errechneten $CH_4$-Produktionsraten aus zugeführtem $H_2$ liegen und daher aus dem Abbau von organischem Substrat stammen müssen (Daten nicht abgebildet).

Die Raumbelastung ist mit ca. 3 kg oTS/m$^3$/d bei beiden Reaktoren vergleichbar. Allerdings unterscheidet sich die Zugabe an Rohfasern deutlich. Währen bei Bioreaktor A über die Dickschlempe nur etwa 0,14 kg Rohfaser/m$^3$/d zugeführt wurden, waren es bei Bioreaktor B über das Stroh mit etwa 1,2 kg Rohfaser/m$^3$/d deutlich mehr.

Bioreaktor B zeigte eine deutlich höhere Leistungsfähigkeit bei der in-situ Methanisierung von $H_2$ und $CO_2$. Selbst bei hohen $H_2$-Zufuhrraten von 0,197 Nm$^3$/m$^3$/h wurden niedrige $H_2$-Ausgangsraten von ca. 0,014 Nm$^3$/m$^3$/h gemessen. Damit ergibt sich nach Gleichung (2) eine $CH_4$-Produktionsrate aus zugeführtem $H_2$ von ca. 0,046 Nm$^3$/m$^3$/h. Bei Bioreaktor A dagegen wurden trotz deutlich kleineren $H_2$-Zufuhrraten von max. nur 0,081 Nm$^3$/m$^3$/h höhere $H_2$-Aus-gangsraten als bei Bioreaktor B von 0,048 Nm$^3$/m$^3$/h gemessen. Das entspricht einer $CH_4$-Produktionsrate aus zuge-führtem $H_2$ bei Bioreaktor A von 0,008 Nm$^3$/m$^3$/h.

**Literaturverzeichnis**

Schiraldi C., De Rosa M. (2014) Mesophilic Organisms. In: Drioli E., Giorno L. (eds) Encyclopedia of Membranes. Springer, Berlin, Heidelberg. https://doi.org/10.1007/978-3-642-40872-4_1610-2

**Patentansprüche**

**1.**    Verfahren zur biologischen in-situ Methanisierung von $CO_2$ und $H_2$ in einem Bioreaktor, **dadurch gekennzeichnet, dass**

a) dem Bioreaktor organisches Substrat zugeführt wird und mindestens ein Teil des organischen Substrates von Mikroorganismen zu Biogas umgewandelt wird,
b) der Bioreaktor bei 20 - 45 °C betrieben wird,
c) dem Bioreaktor $H_2$ zugeführt wird und mindestens ein Teil des $H_2$ zusammen mit $CO_2$ von Mikroorganismen zu Methan umgewandelt wird,
d) Produktgas den Bioreaktor verlässt und einer Produktgas-Aufbereitung zugeführt wird, in der mindestens ein Teil des im Produktgas befindlichen $CO_2$ abgetrennt und in den Bioreaktor zurückgeführt wird, wobei die Konzentration von $CO_2$ im Produktgas einen Wert von 5 % (v/v) bezogen auf das Produktgas ohne den Wasseranteil, nicht unterschreitet.

**2.**    Verfahren nach Anspruch 1, **dadurch gekennzeichnet, dass** mindestens 0,016 Nm$^3$ $CO_2$ pro m$^3$ Bioreaktorvolumen pro Stunde aus dem Produktgas abgetrennt und zurück in den Bioreaktor geführt wird.

**3.**    Verfahren nach Anspruch 1, **dadurch gekennzeichnet, dass** die Konzentration von $CO_2$ im Produktgas einen Wert von 10 % (v/v), besonders bevorzugt 40 % (v/v), bezogen auf das Produktgas ohne den Wasseranteil, nicht unterschreitet.

**4.**    Verfahren nach Anspruch 1, **dadurch gekennzeichnet, dass** der Bioreaktor bei einer Raumbelastung von mindestens 2,5 kg oTS/m$^3$/d, besonders bevorzugt mindestens 3,0 kg oTS/m$^3$/d betrieben wird.

**5.** Verfahren nach Anspruch 1, **dadurch gekennzeichnet, dass** dem Bioreaktor mindestens 0,15 kg Rohfaser pro $m^3$ Bioreaktorvolumen pro Tag, bevorzugt 0,2 kg Rohfaser/$m^3$/d, besonders bevorzugt 1,0 kg Rohfaser/$m^3$/d zugeführt werden.

**6.** Verfahren nach Anspruch 1, **dadurch gekennzeichnet, dass** die $H_2$-Zufuhr mindestens 0,017 $Nm^3/m^3$/h, bevorzugt 0,125 $Nm^3/m^3$/h, besonders bevorzugt 0,167 $Nm^3/m^3$/h beträgt.

**7.** Verfahren nach Anspruch 1, **dadurch gekennzeichnet, dass** durch die Menge an zugeführtem $CO_2$ der pH-Wert im Bioreaktor auf unter 8,2 eingestellt wird.

**8.** Verfahren nach Anspruch 1, **dadurch gekennzeichnet, dass** der $NH_4$-N Gehalt des Bioreaktors durch die Zufuhr einer ammoniumarmen Flüssigkeit und/oder durch die Auswahl des organischen Substrats auf unter 6000 mg/kg geregelt wird.

**9.** Verfahren nach Anspruch 8, **dadurch gekennzeichnet, dass** es sich bei der ammoniumarmen Flüssigkeit um Ablauf aus dem Bioreaktor handelt, dessen $NH_4$-N Gehalt über eine Ammoniumstrippung verringert wurde.

**10.** Verfahren nach Anspruch 1, **dadurch gekennzeichnet, dass** der Bioreaktor der erste Bioreaktor in einer Kaskade von Bioreaktoren ist.

**11.** Verfahren nach Anspruch 1, **dadurch gekennzeichnet, dass** die dem Bioreaktor zugeführten Gase nahe am Boden des Bioreaktors zugeführt werden.

**12.** Verfahren nach Anspruch 1, **dadurch gekennzeichnet, dass** sich im Gasraum des Bioreaktors nur ein geringer Überdruck von weniger als 150 mbar befindet.

Ethanol-anlage

Bioreaktor

Ablaufaufbereitung

Produktgas

Ablauf

Nebenprodukte Ablaufaufbereitung

$CO_2$

Schlempefeststoff

Dickschlempe

$H_2$ aus Elektrolyse

ammoniumarme Prozessflüssigkeit

Fig. 1

Ethanol-anlage

Schlempefeststoff

Dickschlempe

Bioreaktor

$H_2$ aus Elektrolyse

$CO_2$

Produktgas

Ablauf

ammoniumarme Prozessflüssigkeit

Produktgas-Aufbereitung

aufbereitetes Produktgas

Ablaufaufbereitung

Nebenprodukte Ablaufaufbereitung

Fig. 2

14

Fig. 3

Fig. 4 (a) Dickschlempe

Fig. 4 (b) Stroh

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2771472 B1
- EP 2586868 B1
- DE 102012112889 A1
- DE 102012112889
- EP 2586868 A2

**In der Beschreibung aufgeführte Nicht-Patentliteratur**

- Die Untersuchung von Futtermitteln. VDLUFA Verlag, vol. III
- Mesophilic Organisms. **SCHIRALDI C. ; DE ROSA M.** Encyclopedia of Membranes. Springer, 2014